# EUROPÄISCHE PATENTSCHRIFT

(11) **EP 3 017 782 B1**
(45) Veröffentlichungstag und Bekanntmachung des Hinweises auf die Patenterteilung: **23.10.2019**
(21) Anmeldenummer: 15192669.8
(22) Anmeldetag: 03.11.2015
(51) Int. Cl.: A61B 34/30, B25J 9/12, A61B 90/00, A61B 90/50, A61B 90/57, B25J 18/00

(54) **OPERATIONS-ASSISTENZ-SYSTEM**
OPERATION ASSISTANCE SYSTEM
SYSTEME D'ASSISTANCE A L'OPERATION

(30) Priorität: 05.11.2014 DE 102014116103
(43) Veröffentlichungstag der Anmeldung: 11.05.2016
(73) Patentinhaber: Aktormed GmbH, 93092 Barbing (DE)
(72) Erfinder: Kraus, Peter, 95698 Neualbenreuth (DE); Geiger, Robert, 94526 Metten (DE)
(74) Vertreter: Glück Kritzenberger Patentanwälte PartGmbB

(56) Entgegenhaltungen:
- EP-A2- 2 246 006
- DE-A1-102007 019 363
- JP-A- H10 272 143
- US-A- 4 250 762
- US-A- 5 815 640
- US-A1- 2012 143 048
- US-A1- 2014 251 043
- US-B1- 6 211 591

## Beschreibung

Die Erfindung bezieht sich auf ein Operations-Assistenz-System gemäß dem Oberbegriff des Patentanspruches 1.

Operations-Assistenz-Systeme, insbesondere zur Unterstützung von medizinischen Eingriffen oder Operationen sind hinlänglich bekannt. Derartige Systeme werden beispielsweise zur Führung von Hilfsinstrumenten, wie z.B. Kamerasystemen usw. verwendet.

Aus der DE 10 2007 019363 A1 ist beispielsweise ein Operations-Assistenz-System zur Führung von chirurgischen bzw. medizinischen Werkzeugen oder Instrumenten bekannt, mittels dem beispielsweise ein eine Kameraeinheit aufweisendes Endoskop gesteuert geführt wird. Das Operations-Assistenz-System weist hierzu eine gesteuerte Roboterkinematik auf, mittels der ein an dessen freien Ende montiertes chirurgisches bzw. medizinisches Werkzeug oder Instrument im dreidimensionalen Raum gesteuert bewegbar ist. Die Roboterkinematik umfasst beispielsweise wenigstens eine Tragsäule, wenigstens einen ersten und zweiten Roboterarm und wenigstens einen die Instrumentenhalterung aufnehmenden Instrumententräger. Zum Antrieb der Roboterkinematik um mehrere Schwenkachsen sind mehrere hydraulische Antriebseinheiten vorgesehen, die durch entsprechende Zu- oder Abführung eines fluiden Mediums ansteuerbar sind. Nachteilig weisen derartige hydraulische Antriebseinheiten ein Driftverhalten auf, d.h. aufgrund lediglich geringfügiger Druckschwankungen in den hydraulischen Antriebseinheiten kann es zu einer Verschiebung des Roboterkinematik über der Zeit kommen, welche besonders nachteilig bei Operations-Assistenz-Systemen zu einer fehlerhaften Positionierung des an einer Instrumentenhalterung montierten chirurgischen bzw. medizinischen Werkzeuges oder Instrumentes führen kann. Diese Abweichung von einer vorgegebenen Soll-Operationsposition gilt es zu vermeiden. Auch ist bei diesem bekannten Operations-Assistenz-System eine Entkopplung der Antriebseinheiten zur manuellen Führung des an der Instrumentenhalterung montierten chirurgischen bzw. medizinischen Werkzeuges oder Instrumentes nachteilig nur beschränkt möglich.

Aus der DE 10 2011 004 370 A1 ist jedoch bereits ein Gelenkarm zur Halterung von medizinischen Instrumenten bekannt, bei dem mindestens eine Antriebseinheit vorgesehen ist, die in einem aktiven Zustand den Gelenkarm positionieren kann und in einem passiven Zustand manuell zurückstellbar ist. Hierbei ist die Antriebseinheit durch manuelle Krafteinwirkung auf ein vom Gelenkarm gehaltenes Instrument bewegbar. Nachteilig weist dieser Gelenkarm keine Roboterkinematik auf, d.h. dadurch ist ein daran montiertes medizinisches Instrument nur bedingt im dreidimensionalen Raum steuerbar. Zum Einsatz für minimal-invasive Operationen, insbesondere zur gesteuerten Führung oder Nachführung einer Kameraeinheit mittels Bedieners ist ein derartiger Gelenkarm jedoch nicht geeignet. US2012/143048, US5815640, US 2014/251043, US4250762 und US6211591 sind zusätzliche relevante Patentdokumente.

Ausgehend davon ist es Aufgabe der Erfindung, ein Operations-Assistenz-System insbesondere auch für medizinische Interventionen oder Operationen aufzuzeigen, welches eine verbesserte Roboterkinematik aufweist, die dauerhaft positionsgenau fixierbar und dem Operateur eine bedienerfreundliche manuelle Verstellbarkeit ermöglicht. Die Aufgabe wird ausgehend von den Merkmalen des Patentanspruches 1 gelöst.

Ein wesentlicher Aspekt des erfindungsgemäßen Operations-Assistenz-System ist darin zu sehen, dass jeder Antriebseinheit eine steuerbare Magnetbremseneinheit zugeordnet ist, über die die Roboterkinematik von den Antriebseinheiten entkoppelbar ist. Erfindungsgemäß werden mittels der steuerbaren Magnetbremseneinheiten die Arme der Roboterkinematik positions- und lagestabil fixiert, d.h. ein durch die Antriebseinheiten hervorgerufene, ungewünschte Verschiebung der Roboterkinematik aus einer vorgegebenen Sollposition wird effektiv vermieden. Weiterhin vorteilhaft wird die Roboterkinematik von den Antriebseinheiten freigeschaltet und ist im freigeschalteten Zustand durch den Operateur manuell bewegbar, insbesondere durch den Operateur schnell und bedienerfreundlich in eine nahezu beliebige Operationsposition bringbar.
Vorteilhaft sind alle steuerbaren Magnetbremseneinheiten näherungsweise gleichzeitig durch Betätigung eines einzigen Bedienelementes aktivierbar bzw. deaktivierbar. In einer bevorzugten Ausführungsvariante sind diese im deaktivierten Zustand stromlos geschaltet und im aktivierten mit Strom beaufschlagt, wodurch der im stromlosen Zustand hergestellte Reibschluss bzw. Kraftschluss zwischen der jeweiligen Antriebseinheit und der Magnetbremseneinheit aufgehoben wird und damit die Antriebseinheit freigeschaltet wird. Besonders bedienerfreundlich kann der Operateur damit die Freischaltung der gesamten Roboterkinematik durch Betätigen eines Bedienelementes veranlassen.

Weiterhin vorteilhat ist die zweite und/oder dritte Antriebseinheit durch eine Linearantriebseinheit gebildet ist, die zur Erzeugung einer entlang der Tragsäule bzw. entlang des ersten Roboterarmes orientierten linearen Antriebsbewegung ausgebildet ist. Hierbei steht zur Umsetzung der jeweils erzeugten linearen Antriebsbewegung in eine Dreh- oder Schwenkbewegung um die zweite und/oder dritte Schwenkachse ein Drehgelenk umfassend ein kurbelwellenartiges Antriebselement mit der jeweiligen Linearantriebseinheit in Wirkverbindung. Erfindungsgemäß ist die Linearantriebseinheit als Spindelmotoreinheit, die beispielsweise eine Motoreinheit, eine Getriebeeinheit und eine Gewindespindel aufweist.

Erfindungsgemäß weist eine Magnetbremseneinheit zumindest eine in einem Gehäuse vorzugsweise verdrehsicher aufgenommene Magnetbremse auf, die eine stirnseitige Reibfläche aufweist. Die mit der Gewindespindel in Wirkverbindung stehende Gewindemutter ist in einer Ausführungsvariante in einer Mutteraufnahme aufgenommen, die im Gehäuse über Lagereinheiten drehbar gelagert ist. Erfindungsgemäß weist die Mutteraufnahme einen stirnseitigen Reibbelag auf, der in eine Reibverbindung mit der stirnseitige Reibfläche der Magnetbremse bringbar ist.

In einer weiteren Ausführungsvariante ist am zweiten Roboterarm mittels eines abgewinkelten Gelenkstücks ein Instrumententräger angeordnet, wobei das abgewinkelte Gelenkstück eine Rastwelle mit mehreren freiendseitigen Rastnasen aufweist, die mit einer am zweiten Roboterarm angeordneten Rastmutter in Wirkverbindung steht. Das abgewinkelte Gelenkstück ist vorzugsweise manuell drehbar um eine vierte Schwenkachse in einer am zweiten Roboterarm angeordneten, hülsenartigen Rastwellenführungseinheit befestigt.
In einer vorteilhaften Ausführungsvariante weist die hülsenartige Rastwellenführungseinheit einen ersten Führungs- bzw. Aufnahmekanalabschnitt zur Aufnahme der Rastwelle und einen daran anschließenden zweiten Führungs- bzw. Aufnahmekanalabschnitt zur verdrehungssicheren Aufnahme der Rastmutter auf. Die Rastwelle ist vorzugsweise mittels zumindest eines Federelementes mit einer Rückstellkraft beaufschlagt, welche in Richtung der Rastmutter wirkt. Hierdurch ergibt sich für den Operateur eine deutlich verbesserte Zugänglichkeit zum Operationsbereich, d.h. Kollisionen zwischen manuell betätigten Operationsinstrumenten und den an der Roboterkinematik angeordneten Instrumententräger können hierdurch problemlos aufgelöst werden.

In einer vorteilhaften Ausführungsform weist die hülsenartige Rastwellenführungseinheit ein Bedienfeld mit mehreren Schalt- oder Bedienelementen aufweist. Das Bedienelement enthält u.a. das Bedienelement zur näherungsweisen gleichzeitigen Betätigung der steuerbaren Magnetbremseneinheiten.

Die Ausdrucke "näherungsweise", "im Wesentlichen" oder "etwa" bedeuten im Sinne der Erfindung Abweichungen vom jeweils exakten Wert um +/-10%, bevorzugt um +/- 5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: beispielhaft in einer perspektivischer Darstellung ein erfindungsgemäß ausgebildetes Operations-Assistenz-System,
- Fig. 2: beispielhaft einen schematischen Längsschnitt durch den ersten Roboterarm der Roboterkinematik,
- Fig. 3: beispielhaft eine perspektivische Schnittdarstellung durch die am zweiten Roboterarm angeordnete Rastwellenführungseinheit und das damit verbundene abgewinkelte Gelenkstück,
- Fig. 4: beispielhaft eine perspektivische Ansicht der Basisstation ohne Basisgehäuse und
- Fig. 5: eine schematische Seitenansicht der Basisstation ohne Basisgehäuse mit einer Teilschnittdarstellung durch die erste Antriebseinheit.

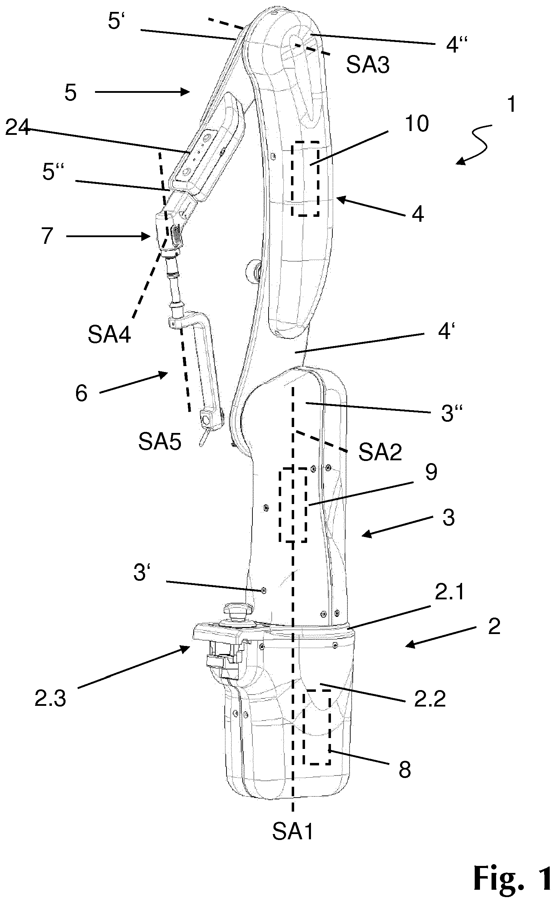

In Figur 1 ist beispielhaft ein erfindungsgemäß ausgebildetes Operations-Assistenz-System 1 für medizinische Eingriffe oder Operationen zur Führung von medizinischen, insbesondere chirurgischen Werkzeugen und Instrumenten dargestellt. Unter einem medizinischen Werkzeug oder Instrument im Sinne der Erfindung wird insbesondere auch eine Kameraeinheit verstanden, welche an einem Endoskop montiert ist und bei einer minimal-invasiven Operation über eine kleinformatige Operationsöffnung ("Trokar") in einen Operationsraum innerhalb eines Patientenkörpers eingeführt wird.

Auch sind andere vergleichbare medizinische Anwendungsmöglichkeiten denkbar, bei denen eine hochgenaue Führung und/oder stabile Fixierung eines medizinischen Werkzeuges oder Instrumentes entlang einer vorgegebenen Führungsbahn und/oder Operationsposition erforderlich sind, ohne dass hierdurch der erfindungsgemäße Gedanke verlassen wird.

Das Operations-Assistenz-System 1 besteht im Wesentlichen aus einer Basiseinheit 2 und einer Roboterkinematik umfassend eine Tragsäule 3, einen ersten und zweiten Roboterarm 4, 5 und ggf. einen Instrumententräger 6 aufweist, wobei der Instrumententräger 6 beispielsweise am zweiten Roboterarm 5 angelenkt ist, und zwar vorzugsweise mittels eines abgewinkelten Gelenkstücks 7. Der Instrumententräger 6 ist beispielsweise zur direkten Aufnahme des medizinischen Werkzeuges oder Instrumentes oder zur indirekten Aufnahme mittels einer Instrumentenhalterung ausgebildet. Der Aufbau der Roboterkinematik ist aus der perspektivischen Darstellung des Operations-Assistenz-Systems 1 gemäß Figur 1 beispielhaft ersichtlich.

Die Basiseinheit 2 umfasst eine Trägerplatte 2.1, ein vorzugsweise mehrteiliges Basisgehäuse 2.2 und zumindest ein Befestigungselement 2.3, mittels dem das Operations-Assistenz-System 1 bzw. die Basiseinheit 2 beispielsweise seitlich an einem Operationstisch befestigbar ist. Im Basisgehäuse 2.2 sind zumindest eine Steuereinheit und ggf. weitere Funktionseinheiten angeordnet (nicht in den Figuren dargestellt).

Die Tragsäule 3 weist einen unteren und oberen Endabschnitt 3', 3" auf. Die Basiseinheit 2 des Operations-Assistenz-System 1 ist mit dem unteren Endabschnitt 3' der Tragsäule 3 der Roboterkinematik mittels einer ersten Antriebseinheit 8 gesteuert schwenkbar um eine erste Schwenkachse SA1 verbunden. Die erste Schwenkachse SA1 verläuft hierbei vertikal zur Aufstellungsebene des Operations-Assistenz-System 1 bzw. Operationsebene bzw. Ebene des Operationstisches.

Ferner weist der erste Roboterarm 4 einen ersten und zweiten Endabschnitt 4', 4" auf, wobei der erste Endabschnitt 4' des ersten Roboterarmes 4 mit dem der Basiseinheit 2 gegenüberliegenden oberen Endabschnitt 3" der Tragsäule 3 mittels einer zweiten Antriebseinheit 9 gesteuert schwenkbar um eine zweite Schwenkachse SA2 und der zweite Endabschnitt 4" des ersten Roboterarmes 4 mit einem ersten Endabschnitt 5' des zweiten Roboterarmes 5 mittels einer dritten Antriebseinheit 10 gesteuert schwenkbar um eine dritte Schwenkachse SA3 verbunden ist. Der zweite Roboterarm 5 weist einen zweiten, dem ersten Endabschnitt 5' gegenüberliegenden Endabschnitt 5" auf, an dem im vorliegenden Ausführungsbeispiel das abgewinkelte Gelenkstücks 7 drehbar um eine vierte Schwenkachse SA4 vorgesehen ist. Das abgewinkelte Gelenkstücks 7 ist zur drehbaren und lösbaren Aufnahme eines Anschlussabschnittes 6' des Instrumententrägers 6 ausgebildet, und zwar um eine fünfte Schwenkachse SA5. Das gegenüberliegende freie Ende 6" des Instrumententrägers 6 bildet eine Instrumentenaufnahme aus.

Im vorliegenden Ausführungsbeispiel sind die erste bis dritte Antriebseinheit 8 - 10 über zumindest eine Steuereinheit vorzugsweise unabhängig voneinander ansteuerbar, um eine Dreh- oder Schwenkbewegung der Roboterkinematik um zumindest die erste bis dritte Schwenkachse SA1 bis SA3 zu bewirken. Vorzugsweise verlaufen die zweite und dritte Schwenkachse SA2, SA3 parallel zueinander, wohingegen die erste Schwenkachse SA1 senkrecht zur zweiten oder dritten Schwenkachse SA2, SA3 orientiert ist. Die erste Antriebseinheit 8 ist vorzugsweise im Basisgehäuse 2.2 aufgenommen und die zweite Antriebseinheit 9 ist entweder seitlich an der Tragsäule 3 angeordnet oder in der Tragsäule 3 integriert bzw. aufgenommen. Analog hierzu ist die dritte Antriebseinheit 10 entweder seitlich am ersten Roboterarm 4 angeordnet oder in diesen integriert bzw. darin aufgenommen.

Die Tragsäule 3 erstreckt sich vertikal, und zwar im Wesentlichen entlang der ersten Schwenkachse SA1, d.h. ist näherungsweise um Ihre eigene Längsachse drehbar ausgebildet. Der erste und zweite Roboterarm 4, 5 erstrecken sich im Wesentlichen ebenfalls entlang einer Geraden, die vorzugsweise senkrecht zur zweiten bzw. dritten Schwenkachse SA2, SA3 verläuft. Im vorliegenden Ausführungsbeispiel weist zumindest der erste Roboterarm 4 eine leichte Krümmung auf.

Erfindungsgemäß sind die zweite und/oder dritte Antriebseinheit 9, 10 durch eine Linearantriebseinheit gebildet, die zur Erzeugung einer entlang der Längsachse der Tragsäule 2 bzw. der ersten Schwenkachse SA1 und/oder des ersten Roboterarmes 4 orientierten linearen Antriebsbewegung AB ausgebildet ist. Zur Umsetzung der hierdurch erzeugten linearen Antriebsbewegung AB in eine Schwenk- oder Drehbewegung um die zweite und/oder dritte Schwenkachse SA2, SA3 steht ein Drehgelenk 11 umfassend ein kurbelwellenartiges Antriebselement 12 mit der jeweiligen Linearantriebseinheit 9, 10 in Wirkverbindung. Erfindungsgemäß ist die Linearantriebseinheit als Spindelmotoreinheit ausgebildet.

Die jeweils als Linearantriebseinheit ausgebildete zweite und/oder dritte Antriebseinheit 9, 10 weisen eine Motoreinheit, vorzugsweise eine Elektromotoreinheit 13 auf, die mit einer Motorbremseneinheit 14 in Wirkverbindung steht. An die Motoreinheit 13 ist eine Getriebeeinheit 15 angeschlossen, über die eine Gewindespindel 16 in Rotation versetzt wird.

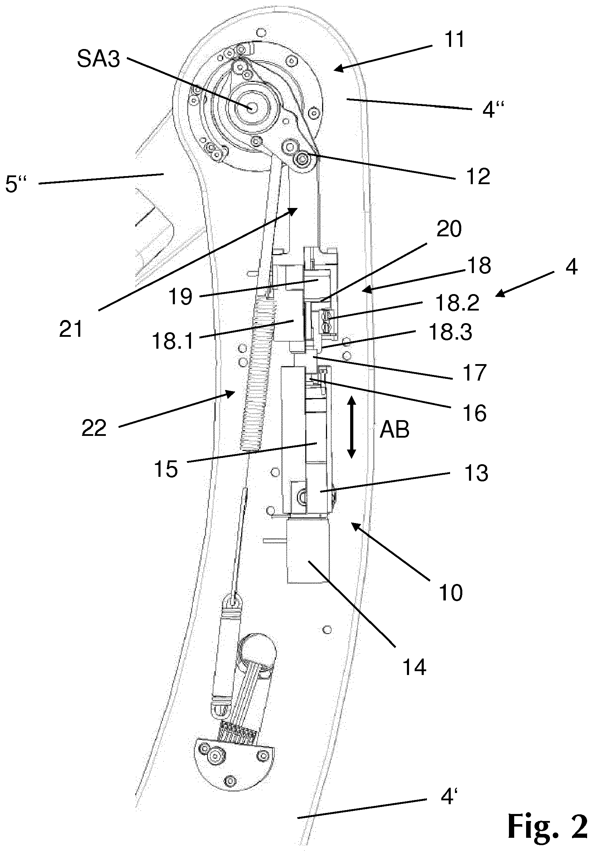

Die Gewindespindel 16 ist über eine Spindelmutter 17 mit einer steuerbaren Magnetbremseneinheit 18 verbunden, die ein Gehäuse 18.1 aufweist, welches sich vorzugsweise konzentrisch um die Gewindespindel 16 erstreckt und in welchem die Gewindespindel 16 mit Ihrem der Linearmotoreinheit 9, 10 gegenüberliegenden freien Ende zumindest abschnittsweise aufgenommen ist. Die Spindelmutter 17 ist über Lagereinheiten 18.2 drehbar im Gehäuse 18.1 gelagert, und zwar mittels eines Mutteraufnehmers 18.3. Vorzugsweise ist der Mutteraufnehmers 18.3 hülsenartig ausgebildet und zumindest teilweise im Gehäuse 18.1 aufgenommen und darin mittels der Lagereinheiten 18.2 drehbar gelagert. Die Gewindespindel 16 erstreckt sich durch den hülsenartigen Mutteraufnehmer 18.3. An dem der Spindelmutter 17 gegenüberliegenden freien stirnseitigen Ende des Mutteraufnehmers 18.3 ist ein eine Reibfläche 20 bildender Reibbelag vorgesehen. An den Mutteraufnehmer 18.3 schließt sich entlang der Gewindespindel 16 dann eine ebenfalls im Gehäuse 18.1 aufgenommene und mit diesem drehfest verbundene Magnetbremse 19 an, die über die stirnseitige Reibfläche 20 in Reibverbindung mit dem Reibbelag des Mutteraufnehmers 18.3 bringbar ist. Bei bestehender Reibverbindung zwischen der Gewindespindel 16 bzw. dem Mutteraufnehmer 18.3 und der Magnetbremse 19 wird die von der Motoreinheit 13 erzeugte Rotationsbewegung in eine lineare Bewegung der Magnetbremseneinheit 18 umgesetzt, die ihrerseits über eine Pleuelstange 21 mit dem freien Kurbelende des kurbelwellenartiges Antriebselement 12 verbunden ist. Über das kurbelwellenartige Antriebselement 12 wird die lineare Antriebsbewegung AR in eine Schwenk- oder Drehbewegung um die zweite oder dritte Schwenkachse SA2, SA3 umgesetzt. Zum Ausgleich des Eigengewichts beispielsweise des in Figur 2 dargestellten zweiten Roboterarms 4 kann beispielsweise eine Federanordnung 22 vorgesehen sein. Die Federanordnung 22 stellt eine elastische Verbindung zwischen dem kurbelwellenartigen Antriebselement 12 und dem gegenüberliegenden ersten Endabschnitt 4' des zweiten Roboterarmes 4 her. Die Federanordnung 22 ist vorzugsweise derart dimensioniert, dass der Operateur im freigeschalteten Zustand der Roboterkinematik diese im Wesentlichen gewichtsneutral verstellen bzw. deren Roboterarme 4, 5 im Raum bewegen kann, und zwar unabhängig von der aktuellen Winkelposition des Drehgelenks 11.

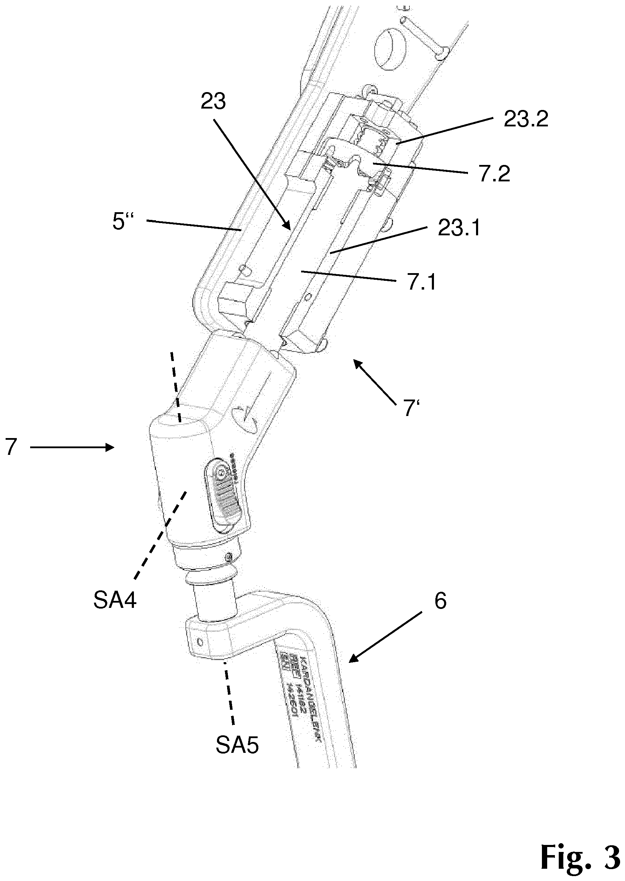

In Figur 3 ist beispielhaft ein perspektivische Schnittdarstellung durch den zweiten Endabschnitt 5"des zweiten Roboterarmes 5 und damit den Anschlussbereich des abgewinkelten Gelenkelements 7 dargestellt. Das abgewinkelte Gelenkelement 7 weist an seinem oberen freien Ende 7' eine Rastwelle 7.1 und eine daran anschließende Rastmutter 7.2 auf. Die Rastwelle 7.1 ist um die vierte Schwenkachse SA4 drehbar im zweiten Endabschnitt 5"des zweiten Roboterarmes 5 aufgenommen, wobei die Rastmutter 7.2 verdrehungssicher im zweiten Endabschnitt 5"des zweiten Roboterarmes 5 angeordnet ist. Zur Aufnahme der Rastwelle 7.1 und der Rastmutter 7.2 ist beispielsweise seitlich am zweiten Endabschnitt 5"des zweiten Roboterarmes 5 eine hülsenartige Rastwellenführungseinheit 23 angeordnet, die einen ersten Führungs- bzw. Aufnahmekanalabschnitt 23.1 zur Aufnahme der Rastwelle 7.1 und einen daran anschließenden zweiten Führungs- bzw. Aufnahmekanalabschnitt 23.2 zur Aufnahme der Rastmutter 7.2 umfasst. Der Durchmesser des ersten Führungs- bzw. Aufnahmekanalabschnittes 23.1 ist kleiner als der Durchmesser des zweiten Führungs- bzw. Aufnahmekanalabschnitt 23.2, so dass sich im Übergangsbereich zwischen den genannten Abschnitten 23.1, 23.2 ein Anlageprofil für die Rastmutterelement 7.2 bildet. Die verdrehungssicher im zweiten Führungs- bzw. Aufnahmekanalabschnitt 23.2 aufgenommene Rastmutter 7.2 weist mehrere um die vierte Schwenkachse SA4 annähernd gleich verteilte Rastöffnungen auf, in die die freiendseitigen Rastnasen der Rastwelle 7.1 eingreifen und somit eine verdrehungssichere Verbindung zwischen dem abgewinkelten Gelenkelement 7 und der Rastwellenführungseinheit 23 am zweiten Roboterarm 5 bilden. Vorzugsweise wird die Rastwelle 7.1 mittels eines Federelementes mit einer Rückstellkraft beaufschlagt, welche in Richtung der Rastmutter 7.2 wirkt und damit die freiendseitigen Rastnasen in die Rastöffnungen der Rastmutter 7.2 drückt. Durch entsprechendes manuelles Herausziehen der Rastwelle 7.1 entgegen der Rückstellkraft aus der hülsenartige Rastwellenführungseinheit 23 wird die Rastverbindung gelöst und der Rotationswinkel um die vierte Schwenkachse SA4 zwischen dem zweiten Roboterarm 5 und dem abgewinkelten Gelenkelement 7 kann entsprechend den vorgegebenen Rastpositionen eingestellt werden.

Gemäß einer Ausführungsvariante der Erfindung kann an der Oberseite der hülsenartigen Rastwellenführungseinheit 23 ein Bedienfeld 24 mit mehreren Schalt- oder Bedienelementen angeordnet sein, über welche der Operateur einzelne oder alle Magnetbremseneinheit 18 der Linearmotoreinheiten 9, 10 derart schalten kann, dass diese eine Entkopplung der Roboterkinematik von den Antriebseinheiten 9, 10 bewirken und die Roboterkinematik von den Antriebseinheiten 9, 10 "freigeschaltet" ist. Im "freigeschalteten" Zustand ist die Roboterkinematik manuell durch den Operateur frei bewegbar und kann somit schnell und einfach an eine neue gewünschte Operationsposition bewegt werden ohne das hierdurch ein über die Antriebseinheiten 8 bis 10 gesteuertes Verfahren der Roboterkinematik erforderlich ist. Im nicht "freigeschalteten" bzw. "gesperrten" Zustand wird die Roboterkinematik mittels der Magnetbremseneinheiten 18 gesperrt und somit dauerhaft und zuverlässig in der vorgegebenen Position gehalten. Hierdurch wird die Bedienung des Operations-Assistenz-Systems 1 für den Operateur wesentlich erleichtert.

Vorzugsweise sind im "gesperrten" Zustand die Magnetbremseneinheiten 18 stromlos, so dass ein Reibschluss zwischen den Linearmotoreinheiten 9, 10 und der jeweils zugehörigen Magnetbremseneinheit 18, 18* gegeben ist. Bei einer entsprechenden Bestromung der Magnetbremseneinheiten 18, 18*, d.h. eine Aktivierung der Magnetbremseneinheiten 18, 18* durch Beaufschlagung mit elektrischer Energie wird die Reibverbindung bzw. der Reibschluss aufgelöst und die Antriebsverbindung getrennt. Damit sind die Antriebseinheiten 8 bis 10 freigeschaltet und die Roboterkinematik kann durch den Operateur frei im Raum bewegt werden.

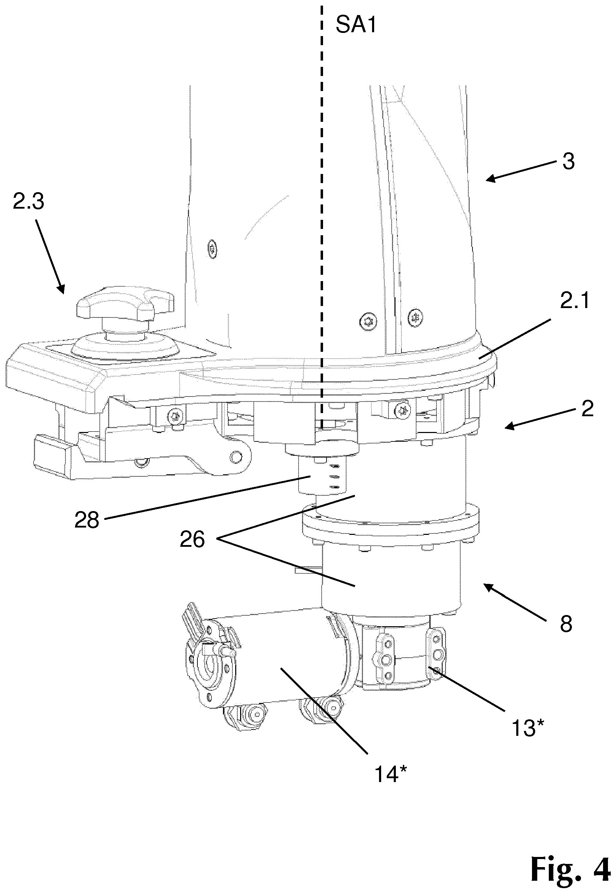

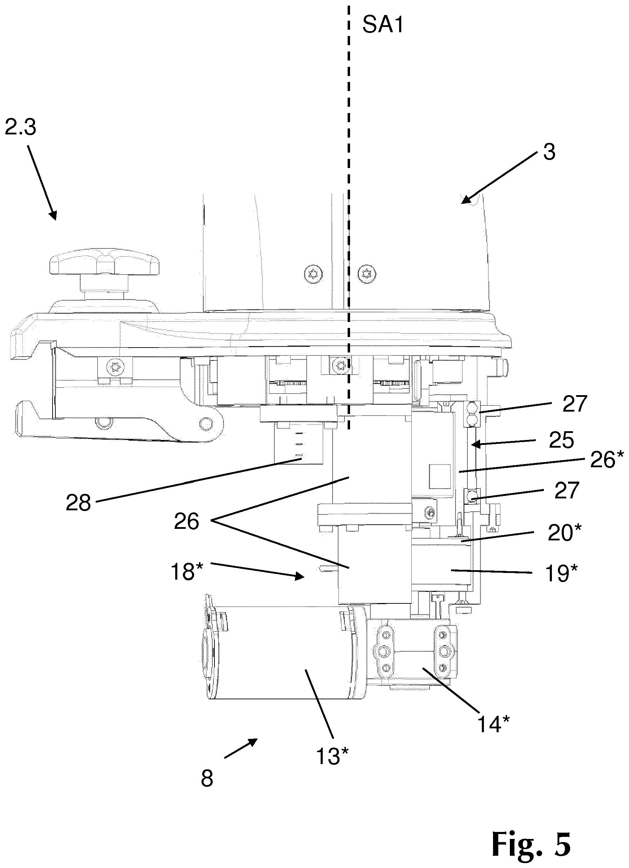

Figur 4 zeigt eine perspektivische Darstellung der Basiseinheit 2 des Operations-Assistenz-Systems 1 ohne das Basisgehäuse 2.1 in einer vergrößerten Ausschnittsdarstellung. Figur 5 zeigt einen Teilschnitt entlang der ersten Schwenkachse SA1 durch die erste Antriebseinheit 8 zum Antrieb der Tragsäule 2 um die erste, vertikale Schwenkachse SA1. Die erste Antriebseinheit 8 weist eine Motoreinheit, vorzugsweise Elektromotoreinheit 13* auf, welche mit einer Getriebeeinheit 14* verbunden ist.

Die Motoreinheit 13* ist über die Getriebeeinheit 14* und eine Magnetbremseneinheit 18* mit einem Planetengetriebeeinheit 25 verbunden, wobei die Magnetbremseneinheit 18* und die Planetengetriebeeinheit 25 in einem gemeinsamem äußeren Gehäuse 26 aufgenommen sind. Die Planetengetriebeeinheit 25 ist selbst wiederum in einem inneren Gehäuse 26* aufgenommen, welches mittels Lagereinheiten 27 im äußeren Gehäuse 26 drehbar um eine parallel zur ersten Schwenkachse SA1 orientierten Drehachse gelagert ist. Die Magnetbremseneinheit 18* weist wiederum eine Magnetbremse 19* auf, die drehfest mit dem äußeren Gehäuse 26 verbunden ist. Am stirnseitigen Ende des inneren Gehäuses 26* ist wiederum ein Reibbelag vorgesehen, welcher eine stirnseitige Reibfläche 20* zur Herstellung eines Reibschlusses bzw. einer Reibverbindung zwischen der Magnetbremseneinheit 18* und der Planetengetriebeeinheit 25 aufweist. Durch stromlos Schalten oder Bestromen der Magnetbremseneinheit 18* ist damit die Motoreinheit 13* antriebsmäßig mit der Planetengetriebeeinheit 25 verbindbar bzw. entkoppelbar. Mittels der Magnetbremseneinheit 18* ist die erste Antriebseinheit 8 damit ebenfalls "freischaltbar" ausgebildet.

Auch kann eine Winkelsensoreinheit 28 zur Erfassung der aktuellen Drehwinkelposition der Tragsäule 3 bezogen auf die erste Schwenkachse SA1 vorgesehen sein.

Zumindest die Roboterarme 4, 5 der Roboterkinematik sind vorzugsweise als Hohlkörper oder als plattenförmige Armelemente ausgebildet und beispielsweise aus einem Werkstoff hergestellt, der eine hohe Stabilität und Belastbarkeit gewährleistet, zur Erzielung einer möglichst geringen Masse ein möglichst geringes spezifisches Gewicht besitzt sowie zugleich auch für bildgebende oder bilderzeugende Medien, beispielsweise für Röntgenstrahlen, Magnetfelder oder elektromagnetische Wellen ein neutraler Werkstoff ist. Als Werkstoff für die als Hohlkörper ausgebildeten Roboterarme 4, 5 wird vorzugsweise ein faserverstärkter, beispielsweise kohlefaserverstärkter Kunststoff verwendet.

### Bezugszeichenliste

- 1: Operations-Assistenz-System
- 2: Basiseinheit
- 2.1: Basisgehäuse
- 2.2: Befestigungselement
- 3: Tragsäule
- 3': unteren Endabschnitt
- 3": oberen Endabschnitt
- 4: erster Roboterarm
- 4': erster Endabschnitt
- 4": zweiter Endabschnitt
- 5: zweiter Roboterarm
- 5': erster Endabschnitt
- 5": zweiter Endabschnitt
- 6: Instrumententräger
- 6': Anschlussabschnittes
- 6": freies Ende
- 7: abgewinkeltes Gelenkstück
- 7': oberer Endabschnitt
- 7.1: Rastwelle
- 7.2: Rastmutter
- 8: erste Antriebseinheit
- 9: zweite Antriebseinheit
- 10: dritte Antriebseinheit
- 11: Drehgelenk
- 12: kurbelwellenartiges Antriebselement
- 13, 13*: Motoreinheit
- 14,: Motorbremseneinheit
- 14*: Getriebeeinheit
- 15: Getriebeeinheit
- 16: Gewindespindel
- 17: Spindelmutter
- 18, 18*: Magnetbremseneinheit
- 18.1: Gehäuse
- 18.2: Lagereinheiten
- 18.3: Mutteraufnehmer
- 19, 19*: Magnetbremse
- 20, 20*: stirnseitige Reibfläche
- 21: Pleuelstange
- 22: Federanordnung
- 23: Rastwellenführungseinheit
- 24: Bedienfeld
- 25: Planetengetriebeeinheit
- 26: äußeres Gehäuse
- 26: inneres Gehäuse
- 27: Lagereinheiten
- 28: Winkelsensor

- AB: lineare Antriebsbewegung
- SA1: erste Schwenkachse
- SA2: zweite Schwenkachse
- SA3: dritte Schwenkachse
- SA4: vierte Schwenkachse
- SA5: fünfte Schwenkachse

## Patentansprüche

1. Operations-Assistenz-System, insbesondere für medizinische Eingriffe oder Operationen, bestehend aus wenigstens einer Basiseinheit (2) und einer daran anschließenden Roboterkinematik umfassend eine Tragsäule (3) sowie zumindest einen ersten und zweiten Roboterarm (4, 5), bei dem ein unterer Endabschnitt (3') der Tragsäule (3) mit der Basiseinheit (2) mittels einer ersten Antriebseinheit (8) gesteuert schwenkbar um eine erste Schwenkachse (SA1), ein erster Endabschnitt (4') des ersten Roboterarmes (4) mit dem der Basiseinheit (2) gegenüberliegenden oberen Endabschnitt (3") der Tragsäule (3) mittels einer zweiten Antriebseinheit (9) gesteuert schwenkbar um eine zweite Schwenkachse (SA2) und ein erster Endabschnitt (5') des zweiten Roboterarmes (5) mit einem zweiten Endabschnitt (4") des ersten Roboterarmes (4) mittels einer dritten Antriebseinheit (10) gesteuert schwenkbar um eine dritte Schwenkachse (SA3) verbunden ist, wobei die erste bis dritte Antriebseinheit (8 - 10) über zumindest eine Steuereinheit steuerbar sind und die erste Schwenkachse (SA1) senkrecht zur zweiten und dritten Schwenkachse (SA2, SA2) verläuft, wobei jeder Antriebseinheit (8- 10) eine steuerbare Magnetbremseneinheit (18, 18*) zugeordnet ist, über die die Roboterkinematik (3 - 5) von den Antriebseinheiten (8 - 10) entkoppelbar ist und die zumindest eine in einem Gehäuse (18.1, 26) verdrehsicher aufgenommene Magnetbremse (19, 19*) mit einer stirnseitigen Reibfläche (20, 20*) aufweist, wobei die zweite und/oder dritte Antriebseinheit (9, 10) durch eine als Spindelmotoreinheit ausgebildete Linearantriebseinheit gebildet ist, die eine Motoreinheit (13), eine Getriebeeinheit (15) und eine Gewindespindel (16) aufweist, und wobei die Gewindespindel (16) über eine Spindelmutter (17) mit der steuerbaren Magnetbremseneinheit (18, 18*) in Wirkverbindung steht und die Spindelmutter (17) in einer Mutteraufnahme (18) aufgenommen ist, die im Gehäuse (18.1) über Lagereinheiten (18.2) drehbar gelagert ist und die einen stirnseitigen Reibbelag aufweist, der in eine Reibverbindung mit der stirnseitigen Reibfläche (20) der Magnetbremse (19) bringbar ist.

2. Operations-Assistenz-System nach Anspruch 1, **dadurch gekennzeichnet, dass** im entkoppelten Zustand die Roboterkinematik (3 - 5) von den Antriebseinheiten (8 - 10) freigeschaltet ist und/oder dass alle steuerbaren Magnetbremseneinheiten (18, 18*) näherungsweise gleichzeitig durch Betätigung eines einzigen Bedienelementes aktivierbar bzw. deaktivierbar sind.

3. Operations-Assistenz-System nach Anspruch 1 oder 2, **dadurch gekennzeichnet, dass** die Spindelmotoreinheit zur Erzeugung einer entlang der Tragsäule (3) bzw. entlang des ersten Roboterarmes (4) orientierten linearen Antriebsbewegung (AR) ausgebildet ist.

4. Operations-Assistenz-System nach Anspruch 3, **dadurch gekennzeichnet, dass** zur Umsetzung der jeweils erzeugten linearen Antriebsbewegung (AR) in eine Dreh- oder Schwenkbewegung um die zweite und/oder dritte Schwenkachse (SA2, SA3) ein Drehgelenk (11) umfassend ein kurbelwellenartiges Antriebselement (12) mit der jeweiligen Linearantriebseinheit (9, 10) in Wirkverbindung steht.

## Claims

1. A surgical operation assistance system, particularly for medical procedures or operations, consisting of at least one base unit (2) and robot kinematics adjoining thereto, comprising a supporting column (3) and at least a first and a second robot arm (4, 5), wherein a lower end section (3') of the supporting column (3) is connected to the base unit (2) so as to be pivotable in controlled manner about a first swivel axis (SA1) by means of a first drive unit (8), a first end section (4') of the first robot arm (4) is connected to the upper end section (3") of the support column (3) opposite the base unit (2) so as to be pivotable in controlled manner about a second swivel axis (SA2) by means of a second drive unit (9), and a first end section (5') of the second robot arm (5) is connected to a second end section (4") of the first robot arm (4) so as to be pivotable in controlled manner about a third swivel axis (SA3) by means of a third drive unit (10), wherein the first to third drive units (8 - 10) are controllable via at least one control unit and the first swivel axis (SA1) extends perpendicularly to the second and third swivel axes (SA2, SA2), wherein a controllable magnetic brake assembly (18, 18*) is assigned to each drive unit (8 - 10), by means of which the robot kinematics (3 - 5) can be decoupled from the drive units (8 - 10), and which comprises at least one magnetic brake (19, 19*) having a frontal friction surface (20, 20*) accommodated in a housing (18.1, 26) in a manner secured against rotation, wherein the second and/or third drive unit (9, 10) is formed by a linear drive unit embodied as a spindle motor unit comprising a motor unit (13), a gearbox unit (15) and a threaded spindle (16), and wherein the threaded spindle (16) is in operative connection with the controllable magnetic brake assembly (18, 18*) via a spindle nut (17) and wherein the spindle nut (17) is accommodated in a nut holder (18) which is pivoted by bearing units (18.2) in the housing (18.1) and which comprises a friction lining on the front face thereof, which can be brought into a frictional connection with the front face friction surface (20) of the magnetic brake (19).

2. Surgical operation assistance system according to claim 1, **characterized in that** in the decoupled state the robot kinematics (3 - 5) is released from the drive units (8 - 10) and/or **in that** all controllable magnetic brake units (18, 18*) can be activated or deactivated at approximately the same time by operating a single control element.

3. Surgical operation assistance system according to claim 1 or 2, **characterized in that** spindle motor unit is designed to generate a linear drive motion (AR) directed along the support column (3) or along the first robot arm (4), respectively.

4. Surgical operation assistance system according to claim 3, **characterized in that** a swivel joint (11) comprising a crankshaft-like drive element (12) is operatively connected to the respective linear drive unit (9, 10) in order to convert the linear drive motion (AR) generated in each case into a rotary or swivelling motion about the second and/or third swivel axis (SA2, SA3).

## Revendications

1. Système d'assistance chirurgicale, destiné notamment à des interventions ou opérations médicales, constitué d'au moins une unité de base (2) et d'une cinématique robotique qui s'y raccorde, comprenant une colonne porteuse (3), ainsi qu'au moins un premier et un deuxième bras de robot (4, 5), sur lequel une partie d'extrémité inférieure (3') de la colonne porteuse (3) est reliée avec l'unité de base (2), de manière à pouvoir pivoter de manière contrôlée au moyen d'une première unité d'entraînement (8) autour d'un premier axe de pivotement (SA1), une première partie d'extrémité (4') du premier bras de robot (4) est reliée avec la partie d'extrémité supérieure (3") de la colonne porteuse (3) qui est opposée à l'unité de base (2), de manière à pouvoir pivoter de manière contrôlée au moyen d'une deuxième unité d'entraînement (9) autour d'un deuxième axe de pivotement (SA2) et une première partie d'extrémité (5') du deuxième bras de robot (5) est reliée avec une deuxième partie d'extrémité (4") du premier bras de robot (4), de manière à pouvoir pivoter de manière contrôlée au moyen d'une troisième unité d'entraînement (10) autour d'un troisième axe de pivotement (SA3), dans lequel les première à troisième unités d'entraînement (8 - 10) sont susceptibles d'être contrôlées par l'intermédiaire d'au moins une unité de commande et le premier axe de pivotement (SA1) s'écoule à la perpendiculaire des deuxième et troisième axes de pivotement (SA2, SA2), dans lequel à chaque unité d'entraînement (8 - 10) est associée une unité de frein magnétique (18, 18*) susceptible d'être contrôlée, par l'intermédiaire de laquelle la cinématique robotique (3 - 5) peut être désaccouplée des unités d'entraînement (8 - 10) et qui comporte au moins un frein magnétique (19, 19*) réceptionné de manière solidaire en rotation dans un carter (18.1, 26) pourvu d'une surface de friction (20, 20*) frontale, la deuxième et/ou troisième unité d'entraînement (9, 10) étant formée d'une unité d'entraînement linéaire conçue sous la forme d'une unité de moteur à broche qui comporte une unité moteur (13), une unité de transmission (15) et une broche filetée (16) et la broche filetée (16) étant en liaison active par l'intermédiaire d'un écrou de broche (17) avec l'unité de frein magnétique (18, 18*) susceptible d'être contrôlée et l'écrou de broche (17) étant réceptionné dans un logement d'écrou (18) qui est logé de manière rotative dans le carter (18.1) par l'intermédiaire d'unités de paliers (18.2) et qui comporte une garniture de friction frontale qui est susceptible d'être amenée en liaison par friction avec la surface de friction (20) frontale du frein magnétique (19).

2. Système d'assistance chirurgicale selon la revendication 1, **caractérisé en ce qu'**en position désaccouplée, la cinématique robotique (3 - 5) est désolidarisée des unités d'entraînement (8 - 10) et/ou **en ce que** toutes les unités de frein magnétique (18, 18*) susceptibles d'être contrôlées sont activables ou désactivables de manière presque simultanée par manoeuvre d'un unique élément de manipulation.

3. Système d'assistance chirurgicale selon la revendication 1 ou 2, **caractérisé en ce que** l'unité de moteur à broche est conçue pour générer un mouvement d'entraînement (AR) linéaire, orienté le long de la colonne porteuse (3) ou le long du premier bras de robot (4).

4. Système d'assistance chirurgicale selon la revendication 3, **caractérisé en ce que** pour convertir le mouvement d'entraînement linéaire (AR) respectivement généré en un mouvement en rotation ou en pivotement autour du deuxième et/ou troisième axe de pivotement (SA2, SA3), une articulation rotative (11) comprenant un élément d'entraînement (12) sous forme d'un vilebrequin est en liaison active avec l'unité d'entraînement linéaire (9, 10) respective.
